## Europäisches Patentamt

## European Patent Office

## Office européen des brevets

(19)

(11) Veröffentlichungsnummer: **0 209 142**
**A2**

(12) # EUROPÄISCHE PATENTANMELDUNG

(21) Anmeldenummer: **86109641.6**

(22) Anmeldetag: **17.07.86**

(51) Int. Cl.⁴: **A 61 K 31/70, A 61 K 31/715, A 61 L 15/01**

(30) Priorität: **17.07.85 DE 3525440**

(43) Veröffentlichungstag der Anmeldung: **21.01.87**
**Patentblatt 87/4**

(84) Benannte Vertragsstaaten: **AT CH DE LI**

(71) Anmelder: **Thiele, Henry, Dr., Ahornstrasse 17, D-7534 Birkenfeld 1 (DE)**

(72) Erfinder: **Thiele, Henry, Dr., Ahornstrasse 17, D-7534 Birkenfeld 1 (DE)**

(74) Vertreter: **Twelmeier, Ulrich, Dipl.Phys., Patentanwälte Dr. Rudolf Bauer Dipl.-Ing.Helmut Hubbuch, Dipl.Phys. Ulrich Twelmeier Westliche Karl-Friedrich-Strasse 29-31, D-7530 Pforzheim (DE)**

(54) **Mittel zur Behandlung von Schürf-, Brand- und sonstigen Wunden.**

(57) Die Erfindung betrifft Mittel aus Mischungen von hochmolekularen Kohlenhydraten und niedermolekularen Kohlenhydraten in verschiedenen Verhältnissen zur Abdeckung von Schürf-, Brand- und sonstigen Wunden. Sie bestehen aus einem Puder, einem Gel und einer anschmiegsamen vernetzten Folie, leicht streubaren Plättchen oder ähnlich geformten streubaren Körpern, sowie aus einem Pflaster. Diese Mittel werden in Verbindung miteinander oder einzeln auf Schürf-, Brand- oder sonstige Wunden aufgebracht und bewirken dort gleichzeitig Schutz, Reinigung und optimale Neubildung der verletzten Hautstellen. Ein grosser Vorteil liegt darin, dass die Neubildung der Gewebezellen auch bei einem Verbandswechsel nicht gestört wird, weil die Wundabdeckung durch ihre aus der erfindungsgemässen Mischung herrührenden gelbindenden Eigenschaften und Vernetzung ohne Abreissen des frisch gebildeten Schorfes und ohne Schmerzen für den Patienten gewechselt werden kann, wodurch die Wunde rascher verheilt als bisher üblich.

ACTORUM AG

- 1 -

## Mittel zur Behandlung von Schürf-, Brand- und sonstigen Wunden.

Die Erfindung betrifft Mittel aus Mischungen hochmolekularer Kohlenhydrate mit niedermolekularen Kohlenhydraten zur Behandlung von Schürf-, Brand- und sonstigen Wunden.

Bisher wurden Salben, Granulate oder Wundabdeckungen verwendet, die meist aus chemisch-synthetischen Stoffen bestehen und die teilweise den Nachteil aufweisen, dass sie auf den Wunden kleben oder schmieren und wieder sehr schwer von der Wunde zu entfernen sind, teilweise nur unter Zerstörung des bereits gebildeten neuen Zellgewebes, was mitunter für den Patienten mit erheblichen Schmerzen verbunden ist.

Es ist bereits bekannt, Kohlenhydrate in Wundabdeckungen einzusetzen, aber nur in Verbindung mit anderen Stoffen, die z.T. chemisch-synthetischen Ursprungs sind. So ist beispielsweise eine Wundabdeckung aus der US-PS 3,972,328 bekannt, die aus einer Masse von Polyisobutylen, quellfähigen Hydrokolloiden, Gelatine und Carboxymethylzellulose besteht, und die aus drei Schichten zusammengesetzt ist und mit Hilfe von Gummielastomeren auf die Wunde geklebt wird.

- 2 -

Ein gravierender Nachteil einer derartigen Wundabdeckung liegt darin, dass sie sich nur schwer von der
verletzten Haut lösen läßt und dass die z.T. synthetische gummiartige Masse wasserunlöslich ist, so dass
beim Abziehen dieser Wundabdeckung von der Wunde immer
das      in und unter dem gebildeten Wundschorf neu
entstandene Hautzellgewebe mit abgerissen wird*), was
die Heilung verlängert und dem Patienten unnötige
Schmerzen bereitet.

*) Literatur:   Ashurst, P.J.: Granulation in chronic leg
                ulcers. A Trial with a new material;
                The Practitioner, September 1975,
                Vol 215, Seite 357 unten.

Durch die vorliegende Erfindung werden diese Nachteile
vermieden, indem die Mittel    ausschließlich aus
hochmolekularen und niedermolekularen Kohlenhydraten
bestehen und keine Eiweißstoffe enthalten.

In der vorliegenden Erfindung hat sich überraschenderweise herausgestellt, dass aus diesen beiden Kohlenhydratarten, den hochmolekularen und den niedermolekularen Kohlenhydraten, die ja mehr dem Lebensmittelsektor zuzuordnen sind, eine Mischung mit völlig neuen
und bisher unbekannten Eigenschaften hergestellt werden
kann. Bei beiden Komponenten handelt es sich um aus
Pflanzen gewonnene, nicht chemisch-synthetisch hergestellte Stoffe, die die im Rahmen der Erfindung vorgegebene Aufgabe in der Kombination hervorragend lösen
können.

- 3 -

Kohlenhydrate bestehen aus Kohlenstoff, Wasserstoff und Sauerstoff und liegen in ihrer einfachsten Form als niedermolekulare Zucker, wie beispielsweise Glucose und Fructose, die als Monosaccharide bekannt sind, vor. Vereinigen sich zwei einfache Zuckermoleküle unter Wasseraustritt, so entsteht das bekannte Disaccharid, die Saccharose oder Rüben- oder Rohrzucker. Vereinigen sich demgegenüber viele der einfachen niedermolekularen Zucker, so bilden sich die hochmolekularen Polysaccharide oder die hochmolekularen Kohlenhydrate, die eine beträchtliche Molekülgröße erreichen können.

Dies sind dann meist zuckerunähnliche Polysaccharide, die nicht mehr süß schmecken, und zu denen Stärke, Glycogen, Zellulose, Lignin, Amylopektin, Agar, Pektin, Dextrin usw. gehören. Sie sind in Wasser entweder unlöslich oder nur kolloidal löslich. Der kolloidale Zustand in einer Lösung ist auf das sehr hohe Molekulargewicht, das über 200 000 betragen kann, zurückzuführen.

In der vorliegenden Erfindung werden nun aus diesen beiden Kohlenhydratarten Mittel zur Behandlung von Schürf-, Brand- und sonstigen Wunden hergestellt, wobei Mischungen, bestehend aus 3 bis 80% hochmolekularen Kohlenhydraten und 97 bis 20 % niedermolekularen Kohlenhydraten, verwendet werden.

Eine wesentliche Eigenschaft dieser Mischungen besteht in ihrer Fähigkeit, in Gegenwart von Flüssigkeit untereinander eine Vernetzung auszubilden, die zu einer geschlossenen Fläche in Art eines Filmes oder einer Folie führt. Es hat sich gezeigt, dass aus der Gruppe der hochmolekularen Kohlenhydrate sich Agar, Zellulose, Amylopektin, Pektin und Stärke in Verbindung mit den niedermolekularen Kohlenhydraten, wie Fructose, Glucose, Saccharose oder deren invertierte Lösungen am besten eignen, um diese wichtige Vernetzung zu erzielen. Besonders bevorzugt ist die Verwendung von Pektin als hochmolekulares Kohlenhydrat. Diese Vernetzung ist wichtig für die Aufnahme und Abgabe von Flüssigkeit. Die Mittel können sich mit viel Flüssigkeit anlösen und mit einem Überschuss an Flüssigkeit auflösen, so dass die Mittel sich zu gegebener Zeit leicht von der Haut durch Abspülen oder Abnehmen entfernen lassen.

Die erfindungsgemäßen Mittel werden beispielsweise eingesetzt in Form eines Puders, eines Gels, einer anschmiegsamen, vernetzten Folie, in Form von leicht streubaren Plättchen oder ähnlich geformten Körpern, sowie in Form von Pflastern.

Für ein Puder zum Aufstreuen auf Schürf-, Brand- oder sonstige Wunden wird vorzugsweise eine Mischung aus 30 bis 70 % hochmolekularen und 70 bis 30 % niedermolekularen Kohlenhydraten verwendet. Am besten eignen sich für diesen Zweck Mischungen aus 35 bis 50 % hochmolekularen und 50 bis 65 % niedermolekularen Kohlenhydraten.

- 5 -

Für ein Gel zum Aufbringen auf Schürf-, Brand- und sonstige Wunden wird am besten der hochmolekulare Anteil der Mischung in Pulverform mit dem niedermolekularen Anteil in überwiegend flüssiger Form im Verhältnis von 3 bis 9 % hochmolekularer zu 97 bis 91 % niedermolekularer Kohlenhydrate verwendet.

Für eine leicht anpassbare, anschmiegsame, vernetzte Folie zum Auflegen auf Schürf-, Brand- und sonstige Wunden wird vorzugsweise eine Mischung aus 20 bis 80 % hochmolekularen und 80 bis 20 % niedermolekularen Kohlenhydraten verwendet. Am besten verwendet man eine Mischung aus 40 bis 60 % hochmolekularen und 60 bis 40 % niedermolekularen Kohlenhydraten.

Für eine Folie nach Art eines Pflasters zum Abdecken von Schürf-, Brand- und sonstigen Wunden wird ebenfalls vorzugsweise eine Mischung aus 20 bis 80 % hochmolekularen und 80 bis 20 % niedermolekularen Kohlenhydraten, am besten aus 40 bis 60 % hoch- und 60 bis 40 % niedermolekularen Kohlenhydraten verwendet.

Für leicht streubare Plättchen oder ähnlich geformte streubare Körper zum Aufstreuen auf Schürf-, Brand- und sonstige Wunden wird ebenfalls vorzugsweise eine Mischung aus 20 bis 80 % hochmolekularen und 80 bis 20 % niedermolekularen Kohlenhydraten, am besten aus 40 bis 60 % hoch- und 60 bis 40 % niedermolekularen Kohlenhydraten verwendet.

Beispiel A
Am Beispiel einer größeren Schürfwunde am Knie, die ja meist stark verschmutzt ist, soll die Anwendung der

- 6 -

erfindungsgemäßen Mittel zur Wundabdeckung dargestellt
werden:

Bei der Behandlung und Versorgung von Wunden kommt es
darauf an, dass die verletzte Hautstelle mechanisch
abgeschirmt wird, dass keine Keime in die Wunde gelangen, dass die Neubildung von Zellen bei der Wundheilung unterstützt und nicht gestört wird und dass
die verschmutzte Wunde gereinigt wird.

Auf die verletzte Stelle wird ein entsprechend großes
Stück der erfindungsgemäßen, leicht anpassbaren, anschmiegsamen,vernetzten Folie, das größer ist als
die verletzte Stelle und die Wundränder um etwa 2 Zentimeter überdeckt, aufgelegt und mit einer Mullbinde oder
Klebstreifen befestigt. Es tritt eine sofortige
Kühlung auf, und es ist kein Brennen oder sonstiger
Schmerz zu beobachten. Nach 24 Stunden kann der Verband
abgenommen werden. Normalerweise tritt kein Anhaften
oder Ankleben am Wundschorf auf. Wenn dies doch der Fall
sein sollte, wird die entsprechende Stelle der Folie
mit sterilem Wasser betupft, und nach kurzer Zeit läßt
sich die Wundabdeckung leicht abnehmen, da die Oberfläche der erfindungsgemäßen Folie durch das Wasser angelöst wird.

Die Wunde und die Wundränder sind sauber und weisen eine
glatte Oberfläche auf, denn das Wundsekret hat die
Oberfläche der anschmiegsamen,vernetzten Folie angelöst und dort ein klebriges Gel gebildet, in das der

- 7 -

Schmutz aus der Wunde eingelagert und dort festgehalten
und schließlich mit dem Abnehmen der Folie leicht entfernt wird.

Nach der Entfernung der Folie von der Wunde kann gegebenenfalls die Haut um die Wunde herum mit dem erfindungsgemäßen Gel bestrichen und die Wundstelle selbst
mit dem erfindungsgemäßen Puder bestreut werden. Nach
einigen Minuten bildet sich über der Wunde und den Wundrändern eine schorfähnliche,trockene Abdeckung, die
nach dem Abtrocknen gegebenenfalls locker mit einer
Mullbinde umwickelt werden kann.
Nach 3 bis 4 Tagen ist die Wunde verheilt, während dies
früher wesentlich länger - manchmal 2 bis 3 Wochen -
dauerte.
Durch die Anwendung der erfindungsgemäßen Mittel ist
gleichzeitig Schutz, Reinigung und optimale Neubildung
der verletzten Hautstelle gegeben.

Bei kleineren Wunden genügt die Anwendung von Gel oder
Puder allein, oder es genügt das Aufbringen der erfindungsgemäßen Folie in der Art eines Pflasters.

Bei Wunden mit unebener Oberfläche und Vertiefungen
verwendet man zweckmässigerweise die erfindungsgemäßen
streubaren Plättchen oder ähnlich geformten streubaren
Körper, wie das Beispiel B zeigt.

- 8 -

Beispiel B

Neben Wunden mit relativ glatter und ebener Oberfläche gibt es auch Wunden, die uneben sind und teilweise kraterförmige Vertiefungen aufweisen. Um auch derartige Wunden mit unebener Oberfläche vollkommen abzudecken, wird die feuchte Wunde mit den erfindungsgemäßen streubaren Plättchen oder ähnlich geformten streubaren Körpern aus einem Streuer oder einer ähnlichen Vorrichtung bestreut, wobei diese aufgestreuten Teilchen durch die Feuchtigkeit der Wunde dort festgehalten werden und sich schuppenartig über- und nebeneinander legen und somit die unebene Wunde in ihrer Gänze abdecken.

Die so abgedeckte Wunde kann dann mit einer Mullbinde umwickelt werden. Die Wundflüssigkeit bringt die Oberfläche der aufgestreuten Teilchen zum Quellen, und diese verbinden sich dann zu einer Art Schorf, unter dem die Wunde ungestört heilen kann. Auch hier wird der in der Wunde vorhandene Schmutz in die Abdeckung eingelagert, so dass er beim Abnehmen der Abdeckung leicht mit entfernt wird.

Diese Entfernung ist sehr leicht, ohne Beeinträchtigung des sich unter der Abdeckung während des Heilvorganges neu gebildeten Gewebes, vorzunehmen, da normalerweise kein Anhaften der Abdeckung an der Wunde auftritt. Wenn dies doch einmal der Fall sein sollte, wird die entsprechende Stelle der Wundabdeckung mit sterilem Wasser betupft, und nach kurzer Zeit läßt sie sich leicht abnehmen.

- 9 -

Herstellungsbeispiele für die erfindungsgemäßen Mittel

Beispiel 1:

Zur Herstellung eines zum Aufstreuen auf Schürf-, Brand-
oder sonstige Wunden besonders geeigneten Puders wird
eine Mischung aus 40 % pulverförmigem Pektin oder einem
anderen vergleichbaren hochmolekularen Kohlenhydrat und
60 % Glucose hergestellt, feinst vermahlen und sterilisiert.

Beispiel 2:

Zur Herstellung eines zum Aufbringen auf Schürf-, Brand-
oder sonstige Wunden besonders geeigneten Gels werden
6 % pulverförmiges Agar oder ein anderes vergleichbares
hochmolekulares Kohlenhydrat, insbesondere Pektin, mit
94 % einer invertierten Saccharoselösung innigst vermischt, bei Temperaturen von über 100°C solange gekocht,
bis ein kolloidales Gel entsteht, das anschließend
hitzesterilisiert und abgefüllt wird.

Beispiel 3:

Zur Herstellung einer leicht anpassbaren, anschmiegsamen,
vernetzten Folie zum Auflegen auf Schürf-, Brand- oder
sonstige Wunden wird eine Mischung aus 60 % Pektin und
40 % feinstgemahlener Saccharose oder einem anderen
vergleichbaren niedermolekularen Kohlenhydrat verwendet,
die zum Erzielen einer gleichen niedrigen Korngröße
noch einmal gemeinsam vermahlen wird, und die anschließend
mit einer sterilen Flüssigkeit zu einer pastenartigen
Masse verarbeitet wird, aus welcher dann durch geeignete
mechanische Vorrichtungen dünne Bahnen hergestellt werden,

- 10 -

die getrocknet, durch Hitze oder auf andere Weise sterilisiert und schließlich steril verpackt werden.

Beispiel 4:

Zur Herstellung einer leicht anpassbaren, anschmiegsamen, vernetzten Folie in der Art eines Pflasters zum Anbringen auf Schürf-, Brand- oder sonstigen Wunden, wird die nach Beispiel 3 gefertigte, die Wunde abdeckende Folie mit einem die Ränder der Folie um einige Zentimeter überragenden, nur auf der Haut um die eigentliche Wunde herum klebenden, geschmeidigen Gewebe oder mit einer Klebefolie verbunden, sterilisiert und verpackt.

Beispiel 5:

Zur Herstellung von streubaren Plättchen oder ähnlich geformten streubaren Körpern zum Aufstreuen auf Schürf-, Brand- und sonstige Wunden wird mit Vorteil eine Mischung aus 50 % nativem Pektin und 50 % feinstgemahlener Saccharose oder einem anderen vergleichbaren, niedermolekularen Kohlenhydrat verwendet, die zum Erzielen einer gleichen geringen Korngröße von einigen µm noch einmal gemeinsam vermahlen und anschließend mit einer sterilen Flüssigkeit zu einer pastenartigen Masse vermischt wird, aus der mittels geeigneter mechanischer Vorrichtungen dünne, am besten zwischen 0,1 mm und 1 mm dicke Bahnen hergestellt werden, aus denen

a) die Plättchen oder ähnlich geformten streubaren Körper des Durchmessers von ca. 1,5 bis 8 mm durch Walzen, Stanzen, Schneiden oder andere mechanische

- 11 -

Vorgänge geformt werden, die anschließend getrocknet und sterilisiert werden, oder

b) die zunächst getrocknet werden, und aus denen dann durch Brechen, Stanzen oder ähnliche mechanische Vorgänge die streubaren Plättchen oder die ähnlich geformten streubaren Körper der entsprechenden Abmessungen hergestellt werden, die anschließend sterilisiert werden.

- 12 -

Patentansprüche:

1. Mittel zur Behandlung von Schürf-, Brand- oder
sonstigen Wunden, bestehend aus einer Mischung von
3 bis 80 Gew.-% hochmolekularer Kohlenhydrate und 97 bis
20 Gew.-% niedermolekularer Kohlenhydrate.

2. Mittel nach Anspruch 1, dadurch gekennzeichnet,
dass die hochmolekularen Kohlenhydrate mit den
niedermolekularen Kohlenhydraten in Gegenwart von
Flüssigkeit vernetzt sind.

3. Mittel nach Anspruch 1 und 2, dadurch gekennzeichnet, dass die hochmolekularen Kohlenhydrate aus
Zellulose, Agar, Stärke, Pektin und/oder Amylopektin und
die niedermolekularen Kohlenhydrate aus Glucose, Fructose,
Saccharose und/oder deren invertierte Lösungen bestehen.

4. Mittel nach einem der Ansprüche 1 bis 3, dadurch
gekennzeichnet, dass es die Form eines auf die verletzte Haut aufzustreuenden Puders aufweist, welches
aus einer Mischung von 30 bis 70 % hochmolekularer
Kohlenhydrate und 70 bis 30 % niedermolekularer Kohlenhydrate besteht.

5. Mittel nach einem der Ansprüche 1 bis 3, dadurch
gekennzeichnet,dass es die Form eines leicht auf
die verletzte Haut aufzutragenden Gels aufweist, welches

- 13 -

aus einer Mischung von 3 bis 9 Gew.-% hochmolekularer Kohlenhydrate und 97 bis 91 Gew.-% niedermolekularer Kohlenhydrate besteht.

6. Mittel nach einem der Ansprüche 1 bis 3, dadurch gekennzeichnet, dass es die Form einer leicht anzupassenden, anschmiegsamen, auf die verletzte Haut auflegbaren Folie aufweist, die aus einer Mischung von 20 bis 80 % hochmolekularer Kohlenhydrate und 80 bis 20 % niedermolekularer Kohlenhydrate besteht, und dass die Bestandteile miteinander vernetzt sind.

7. Mittel nach Anspruch 6, dadurch gekennzeichnet, dass die Folie nach Art eines Pflasters mit einem geschmeidigen, klebenden Gewebe oder einer Klebefolie derart verbunden ist, dass auf die Wunde ausschließlich die aus den Kohlenhydraten gebildete Folie zur Auflage kommt.

8. Mittel nach den Ansprüchen 1 bis 3, dadurch gekennzeichnet, dass es aus kreisförmig oder anders geformten, zum schuppenartigen Abdecken von schwierig zu schützenden und zu bedeckenden, unebenen und unregelmässig vertieften Wunden dienenden, sich mit Sekret oder anderen Flüssigkeiten zu einer geschlossenen Abdeckung miteinander verbindenden, gut streubaren Plättchen oder ähnlich geformten streubaren Körpern mit einem Durchmesser von 1,5 bis 8 mm bei einer Dicke von 0,1 bis 1 mm besteht, dass es aus einer Mischung von 20 bis 80 Gew.-%

- 14 -

hochmolekularer Kohlenhydrate und 80 bis 20 Gew.-% niedermolekularer Kohlenhydrate besteht, und dass die Bestandteile miteinander vernetzt sind.

9. Verfahren zur Herstellung eines Puders nach Anspruch 4 zum Aufstreuen auf Schürf-, Brand- und
sonstige Wunden, dadurch gekennzeichnete, dass die
Mischung feinst vermahlen und sterilisiert wird.

10. Verfahren zur Herstellung eines Gels nach Anspruch 5 zum Aufbringen auf Schürf-, Brand- und
sonstige Wunden, dadurch gekennzeichnet, dass der hochmolekulare Anteil der Mischung in Pulverform mit dem
niedermolekularen Anteil in überwiegend flüssiger Form
vermischt und die Mischung bei hoher Temperatur gekocht
und hitzesterilisiert wird.

11. Verfahren zur Herstellung einer leicht anpassbaren,
auf die verletzte Haut auflegbaren Folie nach Anspruch 6, dadurch gekennzeichnet, dass die Mischung mit
einer sterilen Flüssigkeit zu einer pastenartigen Masse
verarbeitet wird, aus welcher mechanisch dünne Bahnen
gebildet, sterilisiert und steril verpackt werden.

12. Verfahren zur Herstellung einer leicht anpassbaren
Folie in Art eines Pflasters nach den Ansprüchen
6 und 7 , dadurch gekennzeichnet, dass die Folie mit einem
die Folienränder überragenden, geschmeidigen, klebenden
Gewebe oder mit einer Klebefolie verbunden wird.

13. Verfahren zur Herstellung von streubaren Plättchen oder ähnlich geformten, streubaren Körpern nach Anspruch 8, dadurch gekennzeichnet, dass die Mischung der hoch- und niedermolekularen Kohlenhydrate auf eine Teilchengröße von einigem µm gemeinsam vermahlen und die Mischung dann mit einer sterilen Flüssigkeit zu einer pastenartigen Masse vermischt wird, aus welcher mechanisch dünne Bahnen mit einer Dicke von ca. 0,1 mm bis 1 mm gebildet werden,

a) aus denen Plättchen oder ähnlich geformte streubare Körper mit einem Durchmesser von ca. 1,5 mm bis 8 mm durch Walzen, Stanzen, Schneiden oder andere mechanische Vorgänge geformt werden, die anschließend getrocknet und sterilisiert werden, oder

b) die zunächst getrocknet werden, und aus denen dann durch Brechen, Stanzen oder ähnliche mechanische Vorgänge streubare Plättchen oder ähnlich geformte streubare Körper mit einem Durchmesser von ca. 1,5 mm bis 8 mm hergestellt und anschließend sterilisiert werden.